# EUROPÄISCHE PATENTSCHRIFT

(11) **EP 3 589 388 B1**
(45) Veröffentlichungstag und Bekanntmachung des Hinweises auf die Patenterteilung: **01.07.2026**
(21) Anmeldenummer: 18708933.9
(22) Anmeldetag: 22.02.2018
(51) Int. Cl.: B01D 47/12

(54) **VERFAHREN ZUR REINIGUNG DER ABLUFT EINER GRANULIERUNGSANLAGE ZUR HERSTELLUNG EINES HARNSTOFFHALTIGEN GRANULATS**
METHOD FOR CLEANING THE EXHAUST AIR OF A GRANULATING SYSTEM FOR PRODUCING A UREA-CONTAINING GRANULATE
PROCÉDÉ DE PURIFICATION DE L'AIR D'ÉVACUATION D'UN SYSTÈME DE GRANULATION POUR PRODUIRE UN GRANULAT CONTENANT DE L'URÉE

(30) Priorität: 28.02.2017 DE 102017203251
(43) Veröffentlichungstag der Anmeldung: 08.01.2020
(73) Patentinhaber: thyssenkrupp AG, 45143 Essen (DE); thyssenkrupp Uhde Fertilizer Technology GmbH, 44141 Dortmund (DE)
(72) Erfinder: GERNER, Thomas, 44265 Dortmund (DE); RIEKS, Rositsa Marianova, 51381 Leverkusen (DE)
(74) Vertreter: thyssenkrupp Intellectual Property GmbH
(86) Internationale Anmeldenummer: PCT/EP2018/054331
(87) Internationale Veröffentlichungsnummer: WO 2018/158126

(56) Entgegenhaltungen:
- EP-A1- 0 514 902
- EP-A1- 2 192 099
- EP-A1- 2 477 961
- EP-A1- 3 016 731
- FR-A- 1 283 156
- JP-A- 2000 001 466
- US-A- 3 798 021

## Beschreibung

Die vorliegende Erfindung betrifft ein Verfahren zur Reinigung der Abluft einer Granulierungsanlage zur Herstellung eines harnstoffhaltigen Granulats bei dem ein harnstoffhaltiger Staub und Ammoniak enthaltender Gasstrom in einem Waschprozess mit einer Schwefelsäurelösung oder Salpetersäurelösung in Kontakt gebracht wird.

In der Abluft einer Granulierungsanlage zur Herstellung eines harnstoffhaltigen Granulats sind harnstoffhaltige Stäube und Ammoniak enthalten, so dass eine Reinigung der Abluft erforderlich ist, bevor diese in die Umgebung abgegeben werden kann. Bei bekannten Verfahren zur Abluftwäsche in ammoniakhaltigen Gasen fällt eine Ammoniumsulfatlösung mit niedrigem pH-Wert (ca. pH 2-4) an, die in dieser sauren Form nicht unmittelbar weiterverarbeitet werden kann. Eine pH-Wert-Erhöhung ist daher notwendig, um Korrosionsschäden in nachgeschalteten Prozessstufen zu vermeiden.

Aus der EP 2 477 961 B1 ist ein Verfahren zur Rückgewinnung von Harnstoffstaub und Ammoniak aus einem Gasstrom bekannt, bei dem der Gasstrom mit einer Schwefelsäurelösung kontaktiert wird, um so eine Säurelösung von Ammoniumsulfat und Harnstoff zu bilden, wobei dieser Säurelösung Ammoniak hinzugefügt wird. Bei diesem bekannten Verfahren wird ein Gasstrom enthaltend Luft, Harnstoff und Ammoniak in einer Waschvorrichtung mit einer wässrigen Schwefelsäurelösung in Kontakt gebracht, wodurch eine Säurelösung von Harnstoff und Ammoniumsulfat gebildet wird. Diese Säurelösung wird anschließend durch Zugabe von Ammoniak in einer Neutralisiervorrichtung neutralisiert und dann in einer weiteren Vorrichtung aufkonzentriert. In einer Mischvorrichtung kann danach durch Zugabe von Ammoniumsulfat das Verhältnis von Ammoniumsulfat zu Harnstoff auf einen gewünschten Wert erhöht werden und schließlich wird die konzentrierte Schmelze durch Granulierung oder Sprühkristallisation zu einem Feststoff verarbeitet, so dass als Endprodukt ein Feststoffgemisch aus Harnstoff und Ammoniumsulfat erhalten wird.

Die EP 2 192 099 A1 offenbart einen Harnstoffgranulationsprozess, welcher eine saure Abluftbehandlung beinhaltet. Das in der sauren Abluftbehandlung endstehende Ammoniumsulfat kann wieder in den Granulationsprozess zurückgeführt werden.

Die WO 2016/074813 A1 offenbart einen Harnstoffgranulationsprozess umfassend eine Abluftbehandlung mit einer Staubwäsche und einer sauren Abluftwäsche.

Aus JP-A-2000-1466 ist ein Verfahren zur Rückgewinnung und Nutzung von Harnstoffstaub und Ammoniak in Abgasen einer Harnstoffgranulierungsanlage bekannt, bei welchem die in einer Harnstoffgranulierungseinheit anfallenden Abgase zunächst einer Staubwäscheturm zugeführt werden, in dem eine wässrige Harnstofflösung zirkuliert, und anschließend einer sauren Abluftwäsche zugeführt werden, in der mit Säure versetztes Wasser zirkuliert, um Ammoniak zurückzugewinnen, wobei die hierbei erhaltenen Waschlösungen dem Ausgangsmaterial für die Harnstoffgranulierungseinheit zugeführt werden.

Die Aufgabe der vorliegenden Erfindung besteht darin, ein Verfahren zur Reinigung der Abluft einer Granulierungsanlage zur Herstellung eines harnstoffhaltigen Granulats mit den Merkmalen der eingangs genannten Gattung zur Verfügung zu stellen, welches ohne eine externe Nachneutralisation als separatem Prozessschritt auskommt.

Die Lösung der vorgenannten Aufgabe liefert ein Verfahren zur Reinigung der Abluft einer Granulierungsanlage zur Herstellung eines harnstoffhaltigen Granulats der eingangs genannten Art mit den Merkmalen des Anspruchs 1.

Erfindungsgemäß ist vorgesehen, dass der Waschprozess mindestens drei separate aufeinander folgende Waschstufen umfasst, wobei der Gasstrom in einer ersten Waschstufe mit einer ersten schwach sauren Waschlösung gewaschen wird, und der die erste Waschstufe verlassende Gasstrom in einer zweiten Waschstufe mit einer zweiten Waschlösung gewaschen wird, welche einen niedrigeren pH-Wert aufweist als die erste schwach saure Waschlösung, wobei die erste schwach saure Waschlösung eine Harnstoff und Ammoniumsulfat haltige Lösung oder Ammoniumnitrat haltige Lösung mit einem pH-Wert im Bereich von etwa 3,5 bis etwa 6,1 ist . Die Erfindung sieht vor, dass der ersten Waschstufe eine Vorwäsche des Gasstroms in einer Vorwaschstufe vorgeschaltet ist, welche die Aufgabe hat, die in dem Abluftstrom enthaltene Staubmenge wesentlich zu reduzieren. Hierzu kann bevorzugt in der Vorwaschstufe der Gasstrom mit einer verdünnten harnstoffhaltigen Lösung gewaschen werden. Es fällt dabei eine Lösung mit einem erhöhten Gehalt an Harnstoff an, die dann wiederum durch Verdampfen aufkonzentriert werden kann. Der dabei wiedergewonnene Harnstoff kann erneut in der Granulierung zur Herstellung eines harnstoffhaltigen Granulats eingesetzt werden. Diese Vorwäsche hat den Vorteil, dass in den nachfolgenden beiden sauren Waschstufen bereits ein erheblich reduzierter Staubgehalt vorliegt. Die Trennung von Staubwäsche (Vorwaschstufe) und eigentlicher Säurewäsche (erste und zweite Waschstufe) ermöglicht eine Rückführung des im Gasstrom enthaltenden Harnstoffs in den Granulator und eine separate Abtrennung des in der Säurewäsche (erste und zweite Waschstufe) anfallenden Ammoniumsulfats. Das Ammoniumsulfat kann bevorzugt wie in der WO2012/034650 A1 beschrieben, beispielsweise in [0018]-[0022] und [0036]-[0040], in den Granulationsprozess integriert werden.

Zudem setzt Harnstoff bei Kontakt mit säurehaltigen Waschlösungen Isocyanate und Ammoniak (NH₃) frei. Isocyanate zeigen zudem neben ihren bekannten toxischen Eigenschaften eine Tendenz zur Bildung von Aerosolen, welche nur schwierig und aufwendig ausgewaschen werden können und in der Abluft aufgrund ihrer bräunlichen Einfärbung gut erkennbar sind. Beispiele für die Abtrennung von Aerosolen finden sich in der WO2014/094987 A1. Eine weitere Nebenreaktion beinhaltet die Umsetzung von Harnstoff mit Schwefelsäure zu Mono- oder Diureasulfat, welche nicht ohne weiteres in den Stoffkreislauf zurückgeführt werden können.

Eine bevorzugte Weiterbildung der erfindungsgemäßen Aufgabenlösung sieht vor, dass eine in der zweiten Waschstufe anfallende saure Waschlösung in die erste Waschstufe zurückgeführt und dort als die erste schwach saure Waschlösung verwendet wird. Dabei kann insbesondere die erste Waschlösung in der ersten Waschstufe im Kreuzstrom zu dem zu waschenden Gasstrom geführt werden. Ein wichtiger Schritt zur Erzielung einer separaten pH-Wert Korrektur der sauren Waschlösung innerhalb des mehrstufigen Systems zum Waschen der Abluft ist somit bei dieser bevorzugten Variante des erfindungsgemäßen Verfahrens die Gegenstromführung der sauren Waschlösung von einer zweiten Waschvorrichtung zu einer ersten Waschvorrichtung.

Die in der ersten Waschstufe verwendete erste Waschlösung ist nur schwach sauer und hat somit einen höheren pH-Wert als die in der zweiten Waschstufe verwendete stärker saure zweite Waschlösung. Beispielsweise ist die erste schwach saure Waschlösung eine harnstoffhaltige und ammoniumsulfathaltige oder ammoniumnitrathaltige Lösung mit einem pH-Wert im Bereich von etwa 4,3 bis etwa 5,3.

Gemäß dem erfindungsgemäßen Verfahren wird die neutralisierte Ammoniumsulfatlösung oder Ammoniumnitratlösung aus der ersten Waschstufe in die dritte Waschstufe (Vorwaschstufe) geführt und ermöglicht so eine Weiternutzung der erzeugten Ammoniumsulfatlösung.

Vorzugsweise hat die zweite Waschlösung einen pH-Wert von weniger als etwa 2. Beispielsweise kann man konzentrierte Schwefelsäure oder konzentrierte Salpetersäure einerseits und Kondensat aus der Aufkonzentrierungsvorrichtung für die anfallenden harnstoffhaltigen und ammoniumsulfathaltigen Lösungen zuführen, um so die zweite Waschlösung mit einem pH-Wert von vorzugsweise weniger als 2,0 zu erzeugen, mit der der die erste Waschstufe verlassende Gasstrom in der zweiten Waschstufe erneut gewaschen wird.

Bevorzugt wird die erste Waschlösung in der ersten Waschstufe im Kreuzstrom zu dem zu waschenden Gasstrom geführt.

Die durch den Waschvorgang in der ersten Waschstufe anfallende Lösung enthält in der Regel einen gewissen Gehalt an Ammoniumsulfat oder Ammoniumnitrat sowie Spuren von Harnstoff und wird aus der ersten Waschstufe bevorzugt als Lösung mit einem etwa neutralen oder leicht basischen pH-Wert abgeführt. Somit handelt es sich bei der aus der ersten Waschstufe nach dem Waschvorgang abgeführten Lösung bevorzugt um eine ammoniumsulfathaltige oder ammoniumnitrathaltige Lösung mit einem pH-Wert größer 7. Dieser neutrale oder annährend neutrale pH-Wert der Ammoniumsulfatlösung oder Ammoniumnitratlösung hat den Vorteil, dass die Lösung nicht mehr korrosiv ist und unmittelbar weiterverarbeitet werden kann, beispielsweise aufkonzentriert werden kann durch Verdampfung des Wassergehalts. Eine Nachneutralisierung außerhalb des Waschsystems ist nicht mehr erforderlich.

Bevorzugt wird in der Vorwaschstufe der Gasstrom mit einer verdünnten Harnstofflösung gewaschen. Diese kann in der Vorwaschstufe bevorzugt im Kreislauf geführt und anschließend einer Eindampfung und Rückführung in den Granulator zugeführt werden.

Bei dem erfindungsgemäßen Verfahren erfolgt somit durch geeignete Prozessführung die oben genannte pH-Wert Korrektur innerhalb des Waschsystems und es kann somit auf eine externe Nachneutralisation als separatem Prozessschritt verzichtet werden.

Die in der zweiten Waschstufe verwendete stärker saure Waschlösung kann gemäß einer bevorzugten Weiterbildung der Erfindung erzeugt werden, indem der zweiten Waschvorrichtung einerseits konzentrierte Schwefelsäure oder konzentrierte Salpetersäure und andererseits ein Kondensat aus der Aufkonzentrierungsvorrichtung für die anfallenden harnstoffhaltigen und ammoniumsulfathaltigen oder ammoniumnitrathaltigen Lösungen zugeführt wird.

In einer weiteren bevorzugten Ausgestaltung des erfindungsgemäßen Verfahrens wird die neutralisierte Ammoniumsulfatlösung oder Ammoniumnitratlösung aus der ersten Waschstufe abgeführt und beispielsweise einer separaten Verwendung oder Entsorgung zugeführt.

Bevorzugt wird der zweiten Waschstufe ein Kondensat aus einer Aufkonzentrierungsvorrichtung für die anfallenden harnstoffhaltigen und ammoniumsulfathaltigen oder ammoniumnitrathaltigen Lösungen zugeführt. Über die Kondensatzufuhr ist eine Verdünnung und Einstellung der Lösung möglich.

In einer bevorzugten Weiterbildung des erfindungsgemäßen Verfahrens wird der Vorwaschstufe ebenfalls ein Kondensat aus der Aufkonzentrierungsvorrichtung für die anfallenden harnstoffhaltigen und ammoniumsulfathaltigen oder ammoniumnitrathaltigen Lösungen zugeführt, wobei in dieser Vorwaschstufe der harnstoffhaltige Staub bevorzugt mit einer harnstoffhaltigen Lösung ausgewaschen wird. Über die Kondensatzufuhr ist eine Verdünnung der Lösung möglich. Bei dieser Vorwäsche erhöht sich der Harnstoffgehalt der Lösung, da die Waschlösung harnstoffhaltigen Staub aus dem zu waschenden Abluftstrom aufnimmt.

Gegenstand der vorliegenden Erfindung ist weiterhin eine Einrichtung zur Reinigung der Abluft einer Granulierungsanlage zur Herstellung eines harnstoffhaltigen Granulats zur Verwendung in einem Verfahren der zuvor beschriebenen Art, welche wenigstens eine erste Waschvorrichtung umfasst, in der ein harnstoffhaltiger Staub und Ammoniak enthaltender Gasstrom aus der Granulierungsanlage zur Herstellung eines harnstoffhaltigen Granulats mittels einer sauren Waschlösung gewaschen wird, und welche weiterhin wenigstens eine mit der ersten Waschvorrichtung in Wirkverbindung stehende zweite Waschvorrichtung umfasst, in der ein die erste Waschvorrichtung verlassender Gasstrom mit einer weiteren sauren Waschlösung gewaschen wird, welche einen niedrigeren pH-Wert aufweist als die erste saure Waschlösung. Das erfindungsgemäße Konzept sieht somit wenigstens zwei aufeinanderfolgende Waschvorgänge mit jeweils sauren Waschlösungen in jeweils separaten Waschvorrichtungen vor, wobei zunächst mit einer schwach sauren ersten Waschlösung und anschließend mit einer stärker sauren zweiten Waschlösung gewaschen wird. Die Erfindung sieht vor, dass die Einrichtung wenigstens eine dritte Waschvorrichtung umfasst, welche der ersten Waschvorrichtung im Strömungsweg vorgeschaltet ist und in der eine Vorwäsche zum Auswaschen von Staub aus dem Gasstrom stattfindet. Dadurch wird die in den nachgeschalteten beiden sauren Waschstufen aufzunehmende Staubmenge erheblich reduziert.

Dabei umfasst die erfindungsgemäße Einrichtung wenigstens eine Rückführleitung, mittels derer eine in der zweiten Waschvorrichtung anfallende schwach saure Lösung zwecks Verwendung als erste saure Waschlösung in die erste Waschvorrichtung zurückführbar ist. Bei dieser Variante ist es somit besonders vorteilhaft, dass die in der ersten Waschvorrichtung verwendete schwach saure Waschlösung durch den Waschvorgang in der zweiten Waschvorrichtung erzeugt wird und dann von der zweiten Waschvorrichtung in die erste Waschvorrichtung rückgeführt werden kann.

Die erfindungsgemäße Einrichtung zur Reinigung der Abluft einer Granulierungsanlage zur Herstellung eines harnstoffhaltigen Granulats sieht dabei vor, dass diese eine von einer Granulierungsanlage zur Herstellung eines harnstoffhaltigen Granulats ausgehende Abluftleitung umfasst, welche in die dritte Waschvorrichtung führt, wobei weiterhin eine ausgangsseitig von der dritten Waschvorrichtung abführende Gasleitung für in der dritten Waschvorrichtung gereinigte Abluft vorgesehen ist, die von der dritten Waschvorrichtung zur ersten Waschvorrichtung führt und wobei weiterhin eine ausgangsseitig von der ersten Waschvorrichtung abführende Gasleitung für in der ersten Waschvorrichtung gereinigte Abluft vorgesehen ist, die von der ersten Waschvorrichtung zur zweiten Waschvorrichtung führt.

Die Einrichtung zur Reinigung der Abluft einer Granulierungsanlage ist dabei eingerichtet, neutralisierte Ammoniumsulfatlösung oder Ammoniumnitratlösung aus der ersten Waschvorrichtung in die dritte Waschvorrichtung zu führen.

Gegenstand der vorliegenden Erfindung ist weiterhin eine Einrichtung mit der zuvor beschriebenen Einrichtung zur Reinigung der Abluft einer Granulierungsanlage zur Herstellung eines harnstoffhaltigen Granulats, der Granulierungsanlage und einer Konzentrationsvorrichtung, in welcher wenigstens eine von der dritten Waschvorrichtung ausgehende Rückführleitung für eine harnstoffhaltige Lösung vorgesehen ist, welche zu der Konzentrationsvorrichtung führt, in der diese harnstoffhaltige Lösung aufkonzentriert wird, wobei weiterhin Mittel vorgesehen sind, um den dabei gewonnenen Harnstoff in die Granulierungsanlage zur Herstellung eines harnstoffhaltigen Granulats zurückzuführen.

Nachfolgend wird die vorliegende Erfindung anhand eines Ausführungsbeispiels unter Bezugnahme auf die beiliegende Zeichnung näher erläutert. Dabei zeigt:

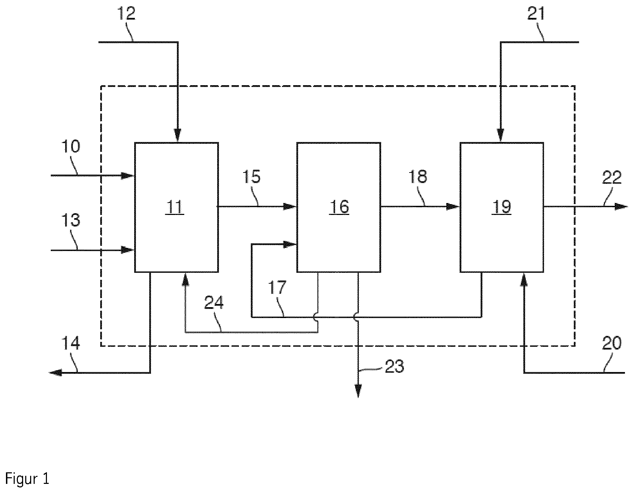

Figur 1 eine schematisch vereinfachte Darstellung einer beispielhaften erfindungsgemäßen Einrichtung zur Reinigung der Abluft einer Granulierungsanlage zur Herstellung eines harnstoffhaltigen Granulats.

Nachfolgend wird unter Bezugnahme auf Figur 1 ein Ausführungsbeispiel der vorliegenden Erfindung näher erläutert. Die Darstellung zeigt ein vereinfachtes Prozessschema, wobei nur die im Rahmen der vorliegenden Erfindung relevanten Anlagenteile dargestellt sind. Über eine Eingangsleitung 10 gelangt die zu reinigende Abluft aus einer Granulierungsanlage zur Herstellung eines harnstoffhaltigen Granulats in eine Waschvorrichtung 11, die als Vorwäsche dient, um den überwiegenden Anteil des harnstoffhaltigen Staubs aus dem Abluftstrom herauszuwaschen. Diese Vorwäsche wird in der vorliegenden Anmeldung auch als dritte Waschvorrichtung 11 bezeichnet. Über eine Leitung 12 kann der Waschvorrichtung 11 ein Kondensat aus der Aufkonzentrierungsvorrichtung für die anfallenden harnstoffhaltigen und ammoniumsulfathaltigen Lösungen zugeführt werden. Als Waschmedium wird in dieser Waschvorrichtung 11 eine harnstoffhaltige Lösung verwendet, die der Waschvorrichtung über die Eingangsleitung 13 zugeführt wird. Bei dem Waschvorgang fällt eine mit Harnstoff angereicherte Lösung an, die beispielsweise Harnstoff in einer Konzentration von 45 % enthalten kann und die über die Ausgangsleitung 14 aus der Waschvorrichtung 11 abgeführt und einer Konzentrationsvorrichtung zugeführt wird, in der das in der Lösung enthaltene Wasser zum größten Teil verdampft wird, um so die hochkonzentrierte harnstoffhaltige Lösung anschließend in die Granulierungsanlage zur Herstellung eines harnstoffhaltigen Granulats zurückgeführt werden kann.

Nach dem Waschvorgang in der Vorwäsche 11 verlässt der größtenteils von Staub befreite Gasstrom die Waschvorrichtung 11 über die Gasleitung 15 und wird nun einer Waschvorrichtung 16 zugeführt, in der der Gasstrom mit einer schwach sauren Lösung gewaschen wird, wobei diese Waschvorrichtung 16 in der vorliegenden Anmeldung als erste Waschvorrichtung bezeichnet wird. Die in dieser ersten Waschvorrichtung verwendete schwach saure Waschlösung, die beispielsweise einen pH-Wert von 4,8 aufweist und die im Wesentlichen eine schwach saure wässrige Lösung von Harnstoff und Ammoniumsulfat oder Ammoniumnitrat ist, wird der Waschvorrichtung 16 über die Eingangsleitung 17 zugeführt. In der Waschvorrichtung 16 wird die schwach saure Waschlösung im Kreuzstrom zu dem über die Leitung 15 eingeleiteten Gasstrom geführt. Diese Eingangsleitung 17 für die Zuführung der Waschlösung in die erste Waschvorrichtung 16 ist dabei wie man Figur 1 entnehmen kann eine Ausgangsleitung aus einer zweiten Waschvorrichtung 19, deren Bedeutung nachfolgend erläutert wird.

Der Gasstrom, der in der ersten Waschvorrichtung 16 gewaschen wurde, verlässt diese erste Waschvorrichtung über die Gasleitung 18 und wird nun in die zweite Waschvorrichtung 19 eingeleitet, in der der Gasstrom erneut gewaschen wird, und zwar mit einer sauren Waschlösung, die stärker sauer ist als die in der ersten Waschvorrichtung 16 verwendete schwach saure Waschlösung. Für diesen Waschvorgang in der zweiten Waschvorrichtung 19 wird zum einen über eine Eingangsleitung 20 konzentrierte Schwefelsäure oder Salpetersäure in die zweite Waschvorrichtung 19 eingeleitet. Weiterhin kann ein Kondensat aus dem Prozess über die Eingangsleitung 21 der zweiten Waschvorrichtung 19 zugeführt werden. In der zweiten Waschvorrichtung 19 erfolgt der saure Waschvorgang beispielsweise mit einer Waschlösung, die einen pH-Wert im Bereich von etwa 1,5 aufweist. Nach diesem erneuten Waschvorgang in der zweiten Waschvorrichtung 19 wird der gereinigte Abluftstrom über die Ausgangsleitung 22 abgeführt und kann im Prinzip in die Umgebung abgegeben werden, da er nahezu staubfrei ist und nahezu kein Ammoniak mehr enthält.

Durch den Waschvorgang des Gasstroms in der zweiten Waschvorrichtung 19 entsteht, da der in diese zweite Waschvorrichtung eingeleitete Gasstrom noch Ammoniak enthält, durch Reaktion mit der über die Leitung 20 eingeleiteten Schwefelsäure oder Salpetersäure Ammoniumsulfat oder Ammoniumnitrat. Da eine teilweise Neutralisation mit dem Ammoniak aus dem Gasstrom in der zweiten Waschvorrichtung stattfindet, wird durch den Waschvorgang eine Lösung erzeugt, die Ammoniumsulfat oder Ammoniumnitrat und Reste von Harnstoff enthält und einen schwach sauren pH-Wert von beispielsweise 4,8 aufweist. Diese schwach saure Harnstoff/Ammoniumsulfat- oder Harnstoff/Ammoniumnitrat - Lösung wird nun über die Ausgangsleitung 17 der zweiten Waschvorrichtung 19 in die erste Waschvorrichtung 16 rückgeführt und dort als Waschlösung verwendet, welche im Kreuzstrom zu dem über die Leitung 15 eingeleiteten Gasstrom geführt wird und zur Gaswäsche in der ersten Waschvorrichtung 16 dient. Die Leitung 17 ist somit gleichzeitig die Eingangsleitung für Waschlösung der ersten Waschvorrichtung 16, die von dem Gasstrom zuerst durchströmt wird. Es erfolgt somit in der ersten Waschvorrichtung 16 eine erste Gaswäsche mit einer ersten schwach sauren Lösung und anschließend in der zweiten Waschvorrichtung 19 erfolgt eine zweite Gaswäsche mit einer stärker sauren Waschlösung.

Aus der ersten Waschvorrichtung 16 kann, da dort ein weiterer Neutralisierungsprozess durch Reaktion der schwach sauren Waschlösung mit in dem Gasstrom enthaltenem Ammoniak erfolgt, über die Ausgangsleitung 23 eine im Wesentlichen neutralisierte wässrige Lösung abgeführt werden, die einen Anteil von gelöstem Ammoniumsulfat oder Ammoniumnitrat enthält, die Spuren von Harnstoff enthält und die einen pH-Wert von größer 7 aufweist.

Gemäß der Erfindung wird auch die neutralisierte Ammoniumsulfatlösung oder Ammoniumnitratlösung über die Leitung 24 aus der ersten Waschstufe 16 in eine dritte Waschstufe (Vorwaschstufe 11) geführt.

Der erfindungsgemäße Aufbau umfasst somit drei Waschstufen zur Reinigung der aus dem Granulator freiwerdenden Abluft von Staub (Vorwaschstufe) und zwei folgenden Stufen zur Entfernung von Ammoniak und gleichzeitigen Anhebung des pH-Wertes in einen für die Leitungen weniger korrosiven Bereiches in der ersten und zweiten Waschstufe.

### Bezugszeichenliste

10 Eingangsleitung
11 Vorwäsche (dritte Waschvorrichtung)
12 Leitung für Kondensat
13 Eingangsleitung für harnstoffhaltige Waschlösung
14 Ausgangsleitung für harnstoffhaltige Lösung
15 Gasführung
16 erste Waschvorrichtung
17 Eingangsleitung für Waschlösung
18 Gasführung
19 zweite Waschvorrichtung
20 Eingangsleitung für Schwefelsäure oder Salpetersäure
21 Eingangsleitung für Kondensat
22 Ausgangsleitung für gereinigten Abluftstrom
23 Ausgangsleitung für neutralisierte Ammoniumsulfatlösung oder neutralisierte Ammoniumnitratlösung
24 Ausgangsleitung für neutralisierte Ammoniumsulfatlösung oder neutralisierte Ammoniumnitratlösung

## Patentansprüche

1. Verfahren zur Reinigung der Abluft einer Granulierungsanlage zur Herstellung eines harnstoffhaltigen Granulats, bei dem ein harnstoffhaltiger Staub und Ammoniak enthaltender Gasstrom in einem Waschprozess mit einer Schwefelsäurelösung oder Salpetersäurelösung in Kontakt gebracht wird,
wobei der Gasstrom in einer ersten Waschstufe (16) mit einer ersten schwach sauren Waschlösung gewaschen wird, und
der ersten Waschstufe (16) eine Vorwäsche des Gasstroms in einer Vorwaschstufe (11) vorgeschaltet ist und in der Vorwaschstufe (11) Staub aus dem Gasstrom ausgewaschen wird, **dadurch gekennzeichnet, dass**
der Waschprozess mindestens zwei separate aufeinander folgende saure Waschstufen (16, 19) umfasst und
der die erste Waschstufe (16) verlassende Gasstrom in einer zweiten Waschstufe (19) mit einer zweiten Waschlösung gewaschen wird, welche einen niedrigeren pH-Wert aufweist als die erste schwach saure Waschlösung, wobei die erste schwach saure Waschlösung eine Harnstoff und Ammoniumsulfat haltige Lösung (17) oder Ammoniumnitrat haltige Lösung (17) mit einem pH-Wert im Bereich von etwa 3,5 bis etwa 6,1 ist, und wobei
die in der zweiten Waschstufe (19) anfallende saure Waschlösung in die erste Waschstufe (16) zurückgeführt und dort als die erste schwach saure Waschlösung verwendet wird, wobei die neutralisierte Ammoniumsulfatlösung oder Ammoniumnitratlösung (24) aus der ersten Waschstufe (16) in die dritte Waschstufe, d.h. Vorwäschestufe 11, geführt wird.

2. Verfahren zur Reinigung der Abluft einer Granulierungsanlage zur Herstellung eines harnstoffhaltigen Granulats nach Anspruch 1, **dadurch gekennzeichnet, dass** die erste schwach saure Waschlösung eine harnstoffhaltige und ammoniumsulfathaltige Lösung (17) oder ammoniumnitrathaltige Lösung (17) mit einem pH-Wert im Bereich von etwa 4,3 bis etwa 5,3 ist.

3. Verfahren zur Reinigung der Abluft einer Granulierungsanlage zur Herstellung eines harnstoffhaltigen Granulats nach einem der Ansprüche 1 oder 2, **dadurch gekennzeichnet, dass** die Waschlösung für die zweite Waschstufe (19) einen pH-Wert von weniger als etwa 2 aufweist.

4. Verfahren zur Reinigung der Abluft einer Granulierungsanlage zur Herstellung eines harnstoffhaltigen Granulats nach einem der Ansprüche 1 bis 3, **dadurch gekennzeichnet, dass** die erste Waschlösung in der ersten Waschstufe (16) im Kreuzstrom zu dem zu waschenden Gasstrom geführt wird.

5. Verfahren zur Reinigung der Abluft einer Granulierungsanlage zur Herstellung eines harnstoffhaltigen Granulats nach einem der Ansprüche 1 bis 4, **dadurch gekennzeichnet, dass** aus der ersten Waschstufe (16) eine ammoniumsulfathaltige Lösung oder eine ammoniumnitrathaltige Lösung mit einem pH-Wert im Bereich von mehr als 7 abgeführt wird.

6. Verfahren zur Reinigung der Abluft einer Granulierungsanlage zur Herstellung eines harnstoffhaltigen Granulats nach einem der Ansprüche 1 bis 5, **dadurch gekennzeichnet, dass** in der Vorwaschstufe (11) der Gasstrom mit einer verdünnten Harnstofflösung gewaschen wird.

7. Verfahren zur Reinigung der Abluft einer Granulierungsanlage zur Herstellung eines harnstoffhaltigen Granulats nach einem der Ansprüche 1 bis 6, **dadurch gekennzeichnet, dass** die neutralisierte Ammoniumsulfatlösung oder Ammoniumnitratlösung (23) aus der ersten Waschstufe (16) abgeführt wird.

8. Verfahren zur Reinigung der Abluft einer Granulierungsanlage zur Herstellung eines harnstoffhaltigen Granulats nach einem der Ansprüche 1 bis 6 **dadurch gekennzeichnet, dass** wenigstens der zweiten Waschstufe (19) ein Kondensat aus einer Aufkonzentrierungsvorrichtung für die anfallenden harnstoffhaltigen und ammoniumsulfathaltigen oder ammoniumnitrathaltigen Lösungen zugeführt wird.

9. Verfahren zur Reinigung der Abluft einer Granulierungsanlage zur Herstellung eines harnstoffhaltigen Granulats nach einem der Ansprüche 1 bis 7, **dadurch gekennzeichnet, dass** der Vorwaschstufe (11) ein Kondensat aus einer Aufkonzentrierungsvorrichtung für die anfallenden harnstoffhaltigen und ammoniumsulfathaltigen Lösungen zugeführt wird.

10. Einrichtung zur Reinigung der Abluft einer Granulierungsanlage zur Herstellung eines harnstoffhaltigen Granulats zur Verwendung in einem Verfahren nach einem der Ansprüche 1 bis 9, umfassend
eine Abluftleitung (10) für einen harnstoffhaltigen Staub und Ammoniak enthaltenden Gasstrom aus der Granulierungsanlage zur Herstellung eines harnstoffhaltigen Granulats,
wenigstens eine dritte Waschvorrichtung (11),
eine abführende Gasführung (15), und
wenigstens eine erste Waschvorrichtung, wobei
- die Abluftleitung (10) in die wenigstens eine dritte Waschvorrichtung (11) führt,
- die wenigstens eine dritte Waschvorrichtung (11) der wenigstens einen ersten Waschvorrichtung (16) im Strömungsweg vorgeschaltet ist und in der wenigstens einen dritten Waschvorrichtung (16) eine Vorwäsche zum Auswaschen von Staub aus dem Gasstrom stattfindet,
- die abführende Gasführung (15) ausgangsseitig von der wenigstens einen dritten Waschvorrichtung (11) für in der wenigstens einen dritten Waschvorrichtung gereinigte Abluft vorgesehen ist, die von der wenigstens einen dritten Waschvorrichtung (11) zur wenigstens einen ersten Waschvorrichtung (16) führt, und
- in der wenigstens einen ersten Waschvorrichtung (16) der Gasstrom mittels einer sauren Waschlösung gewaschen wird, und
**dadurch gekennzeichnet, dass**
die Einrichtung wenigstens eine mit der wenigstens einen ersten Waschvorrichtung (16) in Wirkverbindung stehende zweite Waschvorrichtung (19) umfasst, in der ein die wenigstens eine erste Waschvorrichtung (16) verlassender Gasstrom mit einer weiteren sauren Waschlösung gewaschen wird, welche einen niedrigeren pH-Wert aufweist als die erste saure Waschlösung, weiterhin eine ausgangsseitig von der wenigstens einen ersten Waschvorrichtung (16) abführende Gasführung (18) für in der wenigstens einen ersten Waschvorrichtung (16) gereinigte Abluft vorgesehen ist, die von der wenigstens einen ersten Waschvorrichtung (16) zur zweiten Waschvorrichtung (19) führt, und
die Einrichtung wenigstens eine Rückführleitung (17) umfasst, die eingerichtet ist, eine in der zweiten Waschvorrichtung (19) anfallende schwach saure Lösung zwecks Verwendung als erste saure Waschlösung in die wenigstens eine erste Waschvorrichtung (16) zurückzuführen, wobei diese eingerichtet ist, neutralisierte Ammoniumsulfatlösung oder Ammoniumnitratlösung aus der ersten Waschvorrichtung (16) in die dritte Waschvorrichtung (11) zu führen.

11. Einrichtung mit der Einrichtung zur Reinigung der Abluft einer Granulierungsanlage zur Herstellung eines harnstoffhaltigen Granulats nach Anspruch 10, der Granulierungsanlage und einer Konzentrationsvorrichtung, **dadurch gekennzeichnet, dass** wenigstens eine von der wenigstens einen dritten Waschvorrichtung (11) ausgehende Rückführleitung (14) für eine harnstoffhaltige Lösung vorgesehen ist, welche zu der Konzentrationsvorrichtung führt, in der diese harnstoffhaltige Lösung aufkonzentriert wird, wobei weiterhin Mittel vorgesehen sind, um die dabei gewonnene konzentrierte harnstoffhaltige Lösung in die Granulierungsanlage zur Herstellung eines harnstoffhaltigen Granulats zurückzuführen.

## Claims

1. A process for purifying the exhaust air from a granulation plant for producing a urea-containing granulate, in which in a scrubbing process, a gas stream containing a urea-containing dust and ammonia is brought into contact with a sulfuric acid solution or nitric acid solution,
wherein in a first scrubbing stage (16), the gas stream is scrubbed with a first weakly acidic scrubbing solution,
and
in a pre-scrubbing stage (11), a pre-scrubbing of the gas stream is arranged upstream of the first scrubbing stage (16) and in the pre-scrubbing stage (11), dust is scrubbed out of the gas stream,
**characterized in that**
the scrubbing process comprises at least two separate successive acidic scrubbing stages (16, 19) and
in a second scrubbing stage (19), the gas stream leaving the first scrubbing stage (16) is scrubbed with a second scrubbing solution, which has a lower pH than the first weakly acidic scrubbing solution, wherein the first weakly acidic scrubbing solution is a urea and ammonium sulfate containing solution (17) or ammonium nitrate containing solution (17) having a pH in the range from about 3.5 to about 6.1, and wherein
the acidic scrubbing solution arising in the second scrubbing stage (19) is recycled into the first scrubbing stage (16) and used there as the first weakly acidic scrubbing solution,
wherein the neutralized ammonium sulfate solution or ammonium nitrate solution (24) from the first scrubbing stage (16) is passed into the third scrubbing stage, i.e. pre-scrubbing stage 11.

2. The process for purifying the exhaust air from a granulation plant for producing a urea-containing granulate as claimed in claim 1, **characterized in that** the first weakly acidic scrubbing solution is a urea-containing and ammonium sulfate-containing solution (17) or ammonium nitrate-containing solution (17) having a pH in the range from about 4.3 to about 5.3.

3. The process for purifying the exhaust air from a granulation plant for producing a urea-containing granulate as claimed in one of claims 1 or 2, **characterized in that** the scrubbing solution for the second scrubbing stage (19) has a pH of less than about 2.

4. The process for purifying the exhaust air from a granulation plant for producing a urea-containing granulate as claimed in one of claims 1 to 3, **characterized in that** the first scrubbing solution in the first scrubbing stage (16) is run in crosscurrent with the gas stream to be scrubbed.

5. The process for purifying the exhaust air from a granulation plant for producing a urea-containing granulate as claimed in one of claims 1 to 4, **characterized in that** an ammonium sulfate-containing solution or an ammonium nitrate-containing solution having a pH in the range of more than 7 is discharged from the first scrubbing stage (16).

6. The process for purifying the exhaust air from a granulation plant for producing a urea-containing granulate as claimed in one of claims 1 to 5, **characterized in that** in the pre-scrubbing stage (11), the gas stream is scrubbed with a dilute urea solution.

7. The process for purifying the exhaust air from a granulation plant for producing a urea-containing granulate as claimed in one of claims 1 to 6, **characterized in that** the neutralized ammonium sulfate solution or ammonium nitrate solution (23) from the first scrubbing stage (16) is discharged.

8. The process for purifying the exhaust air from a granulation plant for producing a urea-containing granulate as claimed in one of claims 1 to 6 **characterized in that** at least the second scrubbing stage (19) is supplied with a condensate from a concentration apparatus for the arising urea-containing and ammonium sulfate-containing or ammonium nitrate-containing solutions.

9. The process for purifying the exhaust air from a granulation plant for producing a urea-containing granulate as claimed in one of claims 1 to 7, **characterized in that** the pre-scrubbing stage (11) is supplied with a condensate from a concentration apparatus for the arising urea-containing and ammonium sulfate-containing solutions.

10. An installation for purifying the exhaust air from a granulation plant for producing a urea-containing granulate for use in a process as claimed in one of claims 1 to 9, comprising an exhaust air conduit (10) for a gas stream containing a urea-containing dust and ammonia from the granulation plant for producing a urea-containing granulate,
at least one third scrubbing apparatus (11),
an outlet-side gas conduit (15), and
at least one first scrubbing apparatus, wherein
- the exhaust air conduit (10) leads into the at least one third scrubbing apparatus (11),
- the at least one third scrubbing apparatus (11) is arranged upstream of the at least one first scrubbing apparatus (16) in the flow path and in the at least one third scrubbing apparatus (16), a pre-scrubbing for scrubbing out dust from the gas stream takes place,
- the outlet-side gas conduit (15) is provided on the outlet side of the at least one third scrubbing apparatus (11) for exhaust air purified in the at least one third scrubbing apparatus, which leads from the at least one third scrubbing apparatus (11) to the at least one first scrubbing apparatus (16), and
- in the at least one first scrubbing apparatus (16), the gas stream is scrubbed by means of an acidic scrubbing solution, and
**characterized in that**
the installation comprises at least one second scrubbing apparatus (19) in operative connection with the at least one first scrubbing apparatus (16), in which a gas stream leaving the at least one first scrubbing apparatus (16) is scrubbed with a further acidic scrubbing solution, which has a lower pH than the first acidic scrubbing solution,
furthermore, an outlet-side gas conduit (18) on the outlet side of the at least one first scrubbing apparatus (16) is provided for exhaust air purified in the at least one first scrubbing apparatus (16), which leads from the at least one first scrubbing apparatus (16) to the second scrubbing apparatus (19), and
the installation comprises at least one recycling conduit (17), which is configured to recycle a weakly acidic solution arising in the second scrubbing apparatus (19) into the at least one first scrubbing apparatus (16) for use as first acidic scrubbing solution, wherein it is configured to pass neutralized ammonium sulfate solution or ammonium nitrate solution from the first scrubbing apparatus (16) into the third scrubbing apparatus (11).

11. An installation with the installation for purifying the exhaust air from a granulation plant for producing a urea-containing granulate as claimed in claim 10, the granulation plant and a concentration apparatus, **characterized in that** at least one recycling conduit (14) proceeding from the at least one third scrubbing apparatus (11) is provided for a urea-containing solution, which leads to the concentration apparatus, in which this urea-containing solution is concentrated, wherein means are furthermore provided in order to recycle the concentrated urea-containing solution thereby obtained into the granulation plant for producing a urea-containing granulate.

## Revendications

1. Procédé de purification des gaz d'échappement d'une installation de granulation pour la production d'un granulé contenant de l'urée, dans lequel un flux de gaz contenant de la poussière contenant de l'urée et de l'ammoniac est mis en contact, dans un procédé de lavage, avec une solution d'acide sulfurique ou une solution d'acide nitrique,
dans lequel le flux de gaz est lavé dans une première étape de lavage (16) avec une première solution de lavage faiblement acide, et
une pré-lavage du flux de gaz dans une étape de pré-lavage (11) est disposé en amont de la première étape de lavage (16) et dans l'étape de pré-lavage (11) la poussière est lavée hors du flux de gaz,
**caractérisé en ce que**
le procédé de lavage comprend au moins deux étapes de lavage acides séparées successives (16, 19) et le flux de gaz quittant la première étape de lavage (16) est lavé dans une deuxième étape de lavage (19) avec une deuxième solution de lavage, laquelle présente une valeur de pH plus basse que la première solution de lavage faiblement acide, la première solution de lavage faiblement acide étant une solution (17) contenant de l'urée et du sulfate d'ammonium ou une solution (17) contenant du nitrate d'ammonium ayant une valeur de pH dans la plage d'environ 3,5 à environ 6,1, et dans lequel la solution de lavage acide se produisant dans la deuxième étape de lavage (19) est renvoyée dans la première étape de lavage (16) et y est utilisée comme la première solution de lavage faiblement acide, la solution de sulfate d'ammonium neutralisée ou la solution de nitrate d'ammonium (24) provenant de la première étape de lavage (16) étant conduite dans la troisième étape de lavage, c'est-à-dire l'étape de pré-lavage (11).

2. Procédé de purification des gaz d'échappement d'une installation de granulation pour la production d'un granulé contenant de l'urée selon la revendication 1, **caractérisé en ce que** la première solution de lavage faiblement acide est une solution (17) contenant de l'urée et du sulfate d'ammonium ou une solution (17) contenant du nitrate d'ammonium ayant une valeur de pH dans la plage d'environ 4,3 à environ 5,3.

3. Procédé de purification des gaz d'échappement d'une installation de granulation pour la production d'un granulé contenant de l'urée selon l'une des revendications 1 ou 2, **caractérisé en ce que** la solution de lavage pour la deuxième étape de lavage (19) présente une valeur de pH inférieure à environ 2.

4. Procédé de purification des gaz d'échappement d'une installation de granulation pour la production d'un granulé contenant de l'urée selon l'une des revendications 1 à 3, **caractérisé en ce que** la première solution de lavage dans la première étape de lavage (16) est conduite en écoulement croisé par rapport au flux de gaz à laver.

5. Procédé de purification des gaz d'échappement d'une installation de granulation pour la production d'un granulé contenant de l'urée selon l'une des revendications 1 à 4, **caractérisé en ce qu'**une solution contenant du sulfate d'ammonium ou une solution contenant du nitrate d'ammonium ayant une valeur de pH dans la plage de plus de 7 est évacuée de la première étape de lavage (16).

6. Procédé de purification des gaz d'échappement d'une installation de granulation pour la production d'un granulé contenant de l'urée selon l'une des revendications 1 à 5, **caractérisé en ce que** dans l'étape de pré-lavage (11) le flux de gaz est lavé avec une solution d'urée diluée.

7. Procédé de purification des gaz d'échappement d'une installation de granulation pour la production d'un granulé contenant de l'urée selon l'une des revendications 1 à 6, **caractérisé en ce que** la solution de sulfate d'ammonium neutralisée ou la solution de nitrate d'ammonium (23) est évacuée de la première étape de lavage (16).

8. Procédé de purification des gaz d'échappement d'une installation de granulation pour la production d'un granulé contenant de l'urée selon l'une des revendications 1 à 6, **caractérisé en ce qu'**au moins la deuxième étape de lavage (19) est alimentée avec un condensat provenant d'un dispositif de concentration pour les solutions contenant de l'urée et contenant du sulfate d'ammonium ou contenant du nitrate d'ammonium qui se produisent.

9. Procédé de purification des gaz d'échappement d'une installation de granulation pour la production d'un granulé contenant de l'urée selon l'une des revendications 1 à 7, **caractérisé en ce que** l'étape de pré-lavage (11) est alimentée avec un condensat provenant d'un dispositif de concentration pour les solutions contenant de l'urée et contenant du sulfate d'ammonium qui se produisent.

10. Dispositif pour la purification des gaz d'échappement d'une installation de granulation pour la production d'un granulé contenant de l'urée pour une utilisation dans un procédé selon l'une des revendications 1 à 9, comprenant
une conduite de gaz d'échappement (10) pour un flux de gaz contenant de la poussière contenant de l'urée et de l'ammoniac provenant de l'installation de granulation pour la production d'un granulé contenant de l'urée, au moins un troisième dispositif de lavage (11),
une conduite de gaz évacué (15), et
au moins un premier dispositif de lavage, dans lequel
- la conduite de gaz d'échappement (10) conduit dans le au moins un troisième dispositif de lavage (11),
- le au moins un troisième dispositif de lavage (11) est disposé en amont dans le trajet d'écoulement du au moins un premier dispositif de lavage (16) et dans le au moins un troisième dispositif de lavage (16) un pré-lavage pour éliminer la poussière du flux de gaz a lieu,
- la conduite de gaz évacué (15) est prévue en aval du au moins un troisième dispositif de lavage (11) pour les gaz d'échappement nettoyés dans le au moins un troisième dispositif de lavage, qui conduit du au moins un troisième dispositif de lavage (11) vers le au moins un premier dispositif de lavage (16), et
- dans le au moins un premier dispositif de lavage (16) le flux de gaz est lavé au moyen d'une solution de lavage acide, et
**caractérisé en ce que**
le dispositif comprend au moins un deuxième dispositif de lavage (19) en liaison fonctionnelle avec le au moins un premier dispositif de lavage (16), dans lequel un flux de gaz quittant le au moins un premier dispositif de lavage (16) est lavé avec une autre solution de lavage acide, laquelle présente une valeur de pH plus basse que la première solution de lavage acide, en outre une conduite de gaz évacué (18) en aval du au moins un premier dispositif de lavage (16) est prévue pour les gaz d'échappement nettoyés dans le au moins un premier dispositif de lavage (16), qui conduit du au moins un premier dispositif de lavage (16) vers le deuxième dispositif de lavage (19), et
le dispositif comprend au moins une conduite de retour (17), qui est configurée pour renvoyer une solution faiblement acide se produisant dans le deuxième dispositif de lavage (19) aux fins d'utilisation comme première solution de lavage acide dans le au moins un premier dispositif de lavage (16), celui-ci étant configuré pour conduire la solution de sulfate d'ammonium neutralisée ou la solution de nitrate d'ammonium du premier dispositif de lavage (16) dans le troisième dispositif de lavage (11).

11. Dispositif avec le dispositif pour la purification des gaz d'échappement d'une installation de granulation pour la production d'un granulé contenant de l'urée selon la revendication 10, l'installation de granulation et un dispositif de concentration, **caractérisé en ce qu'**au moins une conduite de retour (14) pour une solution contenant de l'urée provenant du au moins un troisième dispositif de lavage (11) est prévue, laquelle conduit vers le dispositif de concentration, dans lequel cette solution contenant de l'urée est concentrée, en outre des moyens étant prévus afin de renvoyer la solution contenant de l'urée concentrée obtenue ainsi dans l'installation de granulation pour la production d'un granulé contenant de l'urée.
